# EUROPEAN PATENT SPECIFICATION

(11) **EP 3 302 474 B1**
(45) Date of publication and mention of the grant of the patent: **17.03.2021**
(21) Application number: 16804318.0
(22) Date of filing: 01.06.2016
(51) Int. Cl.: A61K 9/06, A61K 9/107, A61K 47/44, A61K 47/46, A61P 15/02, A61K 9/00, A61K 45/06, A61K 35/644, A61K 36/61, A61K 31/4535

(54) **COMPOSITIONS FOR TREATMENT OF ATROPHIC VAGINITIS, PERI-AND POST-MENOPAUSAL DYSPAREUNIA, AND/OR OOPHORECTOMIZED FEMALES AND TREATMENT METHODS THEREWITH**
ZUSAMMENSETZUNGEN ZUR BEHANDLUNG VON ATROPHISCHER VAGINITIS, PERI- UND POSTMENOPAUSALER DYSPAREUNIE UND/ODER OOPHOREKTOMIERTEN FRAUEN UND BEHANDLUNGSVERFAHREN DAMIT
COMPOSITIONS POUR LE TRAITEMENT DE LA VAGINITE ATROPHIQUE, LA DYSPAREUNIE PÉRI ET POST-MÉNOPAUSIQUE, ET/OU DE FEMMES OVARIECTOMISÉES ET PROCÉDÉS DE TRAITEMENT AVEC CELLES-CI

(30) Priority: 01.06.2015 US 201562169105 P; 13.11.2015 US 201562255059 P
(43) Date of publication of application: 11.04.2018
(73) Proprietor: Durga Enterprises, LLC, Springboro, OH 45066 (US)
(72) Inventor: CHRISTOPHER, Anastasia M., Springboro, OH 45066 (US)
(74) Representative: Studio Torta S.p.A.
(86) International application number: PCT/US2016/035231
(87) International publication number: WO 2016/196608

(56) References cited:
- WO-A1-2015/030609
- WO-A2-2007/143607
- US-A- 5 980 875
- US-A1- 2008 103 155
- US-A1- 2014 219 929
- US-A1- 2014 219 929

## Description

### RELATED APPLICATIONS

### FIELD

This application relates to compositions for treatment of atrophic vaginitis, peri- and post-menopausal dyspareunia, and/or oopherectomized females prior to menopause, in particular compositions administered into the vagina that contain a selective estrogen receptor modulator, an intracellular carrier, and a cellular anti-inflammatory agent, and treatment methods using the same.

### BACKGROUND

Atrophic vaginitis, peri- and post-menopausal dyspareunia, and/or oopherectomized females prior to menopause can occur in women with the progression of aging or sometimes as a result of medical treatments, such as some cancer treatments. The symptoms related with both of these conditions include pain, itching, vaginal dryness, vaginal bleeding or mild spotting, and urogenital infections. Relief from these symptoms and a return to pleasant, non-painful intercourse are needed for these women.

Treatments using estrogen may be suitable for some women, but are not suitable for women who are at risk of breast, uterine, endometrial, ovarian, or fallopian tube cancer or have had such cancer(s). Accordingly, there is a need for an effective treatment of atrophic vaginitis and/or peri- and post-menopausal dyspareunia that is free of estrogen.

Atrophic vaginitis can be treated with a composition disclosed in WO2007/143607. Further compositions are disclosed in US2014/219929.

### SUMMARY

The present invention relates to a composition according to claim 1.

In one aspect, compositions for topical administration in a urogenital area and/or a vagina are disclosed herein, in particular compositions that are substantially free of estrogen. Such compositions provide a much needed alternative treatment for women who, for medical reasons, should not receive estrogen. The compositions have a therapeutically effective amount of a selective estrogen receptor modulator (SERM), an intracellular carrier for carrying the SERM into a cell, and a therapeutically effective amount of a cellular anti-inflammatory agent. The SERM is one or more of droloxifene, idoxifene, raloxifene, tamoxifen, toremifene, and TAT-59. In one embodiment, the SERM includes at least raloxifene. According to the claimed invention, it is present as about 0.038 mg/ml to about 1.9 mg/ml of the composition.

The intracellular carrier is a natural oil, which, in one embodiment, is selected from the group consisting of almond oil, avocado oil, coconut oil, corn oil, flaxseed oil, mustard oil, olive oil, peanut oil, rice bran oil, soybean oil, walnut oil, and combinations thereof. In another embodiment, the intracellular carrier includes avocado oil, coconut oil, and/or olive oil. The intracellular carrier preferably is present in the composition as about 15% by volume to about 54% by volume of the liquid ingredients.

The cellular anti-inflammatory agent is a medicinal grade honey, for example Manuka honey or a honey from plants of the *Leptospermum* species. It is believed that the methylglyoxal content of honey made from plants of the *Leptospermum* species improves the antibacterial and immunostimulatory properties thereof. The medicinal grade honey is present in the composition as about 30% by volume to about 62% by volume of the total liquid ingredients.

The compositions may include a solute that dissolved the SERM. In another embodiment, the compositions may include a thickening agent, and/or one or more tocopherol and/or tocotrienols that have Vitamin E activity.

In another aspect, the claimed composition is for use in treating atrophic vaginitis, peri- and post-menopausal dyspareunia, and/or oopherectomized females prior to menopause . The method includes providing one of the compositions disclosed herein and topically applying about 2 ml to about 8 ml of the composition to the urogenital area and/or into the vagina daily, every other day, or at least once weekly. Topically applying the composition is performed just prior to a user lying down for at least four hours.

In another aspect, compositions for topical administration in a urogenital area and/or a vagina for treatment of atrophic vaginitis, peri- and post-menopausal dyspareunia, and/or oopherectomized females prior to menopause that are free of estrogen and free of a selective estrogen receptor modulator are also disclosed. The compositions include a therapeutically effective amount of a medicinal grade honey, a therapeutically effective amount of a natural oil, and are free of estrogen and free of a selective estrogen receptor modulator (SERM). The natural oil may be one or more of almond oil, avocado oil, coconut oil, corn oil, flaxseed oil, mustard oil, olive oil, peanut oil, rice bran oil, soybean oil, walnut oil, or other natural oils. The natural oil is preferably about 15% by volume to about 54% by volume of the composition. In one embodiment, the natural oil is avocado oil, coconut oil, and/or olive oil. In another embodiment, the natural oil is a 1:1 mixture of avocado oil and coconut oil. The medicinal grade honey includes Manuka honey, and is about 30% by volume to about 62% by volume of the composition. The composition may include one or more of a thickening agent, and/or one or more tocopherol and/or tocotrienols that have Vitamin E activity.

The features, functions, and advantages that have been discussed can be achieved independently in various embodiments or may be combined in yet other embodiments, further details of which can be seen with reference to the following description.

### DESCRIPTION

Compositions for administration in the vagina are disclosed herein that include a therapeutically effective amount of a selective estrogen receptor modulator (SERM); an intracellular carrier for carrying the SERM into a cell; and a therapeutically effective amount of a cellular anti-inflammatory agent. Since the compositions use a SERM as the therapeutic agent, these compositions can be free of estrogen. As used herein, "free of estrogen" means that the composition includes no estrogen or less than 0.001 mg/ml by weight of an estrogen, more preferably less than 0.0001 mg/ml of an estrogen.

### SERM

The SERM may be one or more of droloxifene, idoxifene, raloxifene, tamoxifen, toremifene, TAT-59 ((E)-4-[1-[4-[2-(dimethylamino)ethoxy]-phenyl]-2-(4-isopropyl) phenyl-1-butenyl]phenyl monophosphate), or derivatives thereof that retain the properties of a selective estrogen receptor modulator. In one embodiment, the SERM is at least raloxifene. In another embodiment, the SERM is only raloxifene. The SERM in total comprises about 0.038 mg/ml of the composition to about 1.9 mg/ml of the composition. In one embodiment, the SERM in total comprises about 0.15 mg/ml of the composition to about 1 mg/ml of the composition. In a preferred embodiment, the SERM in total comprises about 0.3 mg/ml of the composition to about 0.7 mg/ml of the composition.

The SERM may be dissolved in a solute. The solute may be glycerin, propylene glycol, butylene glycol, hexylene glycol or polyethylene glycol of various molecular weight and the like and/or combinations thereof.

### Intracellular Carrier

The intracellular carrier is a natural oil which is preferably selected from the group consisting of almond oil, avocado oil, coconut oil, corn oil, flaxseed oil, mustard oil, olive oil, palm oil, palm kernel oil, peanut oil, rice bran oil, soybean oil, theobroma oil, walnut oil, and combinations thereof. In one embodiment, the intracellular carrier included in the composition has one or more of avocado oil, coconut oil, and olive oil.

The intracellular carrier may be present in the composition, based liquid ingredients, i.e., all ingredients except for the SERM, as about 15% by volume to about 54% by volume of the liquid ingredients. In another embodiment, the intracellular carrier may be present as about 23% by volume to about 38% by volume of the liquid ingredients. One example composition comprises equal amounts of avocado oil and coconut oil.

### Cellular Anti-inflammatory Agent

The cellular anti-inflammatory agent is one that reduces inflammation resulting from a cytokine response, specifically a cytokine response from the release of cytokines from monocytes and macrophages. According to the invention, the cellular anti-inflammatory agent includes a medicinal grade honey, such as a medicinal grade Manuka honey. The medicinal grade honey is present as about 30% by volume to about 62% by volume of the total liquid ingredients. In another embodiment, the medicinal grade honey may be present as about 38% by volume to about 50% by volume of the total liquid ingredients.

### Tocopherols and Tocotrienols

Tocopherols and/or tocotrienols having Vitamin E activity include, but are not limited to, alpha tocopherol, D alpha tocopherol, DL alpha tocopherol, or tocopheryl acetate, alpha, beta, gamma, and delta tocotrienols. The tocopherols and/or tocotrienols may be present as about 1% by volume to about 15% by volume of the total liquid ingredients. In another embodiment, the tocopherols and/or tocotrienols may be present as about 4% by volume to about 8% by volume of the total liquid ingredients.

### Thickening Agent

Optionally, the vaginal compositions may include an effective amount of a thickening agent. The thickening agent may be any one or more of the following: pectin, algin, gum arabic, propylene glycol, methyl cellulose, and carnauba wax. The effective amount is determined based on a desired viscosity that makes the vaginal composition spreadable within the vagina without creating a vaginal discharge that would be excessive and/or undesirable to the end user. The thickening agent may be present as about 1% by volume to about 15% by volume of the total liquid ingredients. In another embodiment, the thickening agent may be present as about 4% by volume to about 8% by volume of the total liquid ingredients.

### Pain Reliever

Optionally, the vaginal compositions may include a pain reliever as about 1% to about 7% by weight of the composition, or more preferably about 1% to about 4% by weight of the composition. The pain reliever may be benzocaine, which may be provided as a powder. The benzocaine may be added to the raloxifene Phase B mixture, as explained in the Examples, and then incorporated into Phase A.

### pH Adjuster

The vaginal compositions may also include a pH adjuster, in particular a pharmaceutically acceptable pH adjuster. The pH adjuster may be an acid, base, or buffer. In one embodiment, the pH adjuster is an acid, such as a weak acid. The weak acid may be but is not limited to, lactic acid, citric acid, ascorbic acid, and the like and combinations thereof. The acid is added to adjust the pH of the composition to within a range of about 4.5 to about 6. In one embodiment, aloe vera juice was added to correct the pH to within the range of about 4.5 to about 6. In another embodiment, additional tocopherols and/or tocotrienols and/or medicinal grade honey were added to adjust the pH.

### Additives

The vaginal compositions may also include other additives or inactive ingredients, such as, but not limited to, purified, sterile water, fillers, preservatives, absorption enhancers, colorants, and emulsifiers. The fillers may include, but are not limited to, titanium dioxide, anhydrous lactose, lactose monohydrate, and magnesium stearate, and combinations thereof. The preservatives may include, but are not limited to, methylparaben, propylparaben, potassium sorbate, benzalkonium chloride, and benzethonium chloride, of which one or more may be present in the vaginal suppository compositions. The absorption enhancers include, but are not limited to crospovidone. The colorants include, but are not limited to, FD&C Blue #3 (alum lake). The emulsifiers include, but are not limited to, povidone, and polysorbate 80.

The compositions for administration in the vagina disclosed herein are effective at soothing and reducing symptoms of atrophic vaginitis. In the compositions, the medicinal grade honey, is a non-irritant to menopausal or atrophic urogenital cells, and decreases the cellular inflammation inherent to atrophic cells so that these cells can begin to heal. Once the atrophic urogenital cells have decreased inflammation, it is possible for the intracellular carrier to facilitate passage of the SERM into the urogenital cells at an intracellular level. The SERMs are typically lipophilic and the intracellular carrier is preferably a natural oil, which increases the bioavailability of the SERM, so that the SERM can intracellularly bind with estrogen receptor sites, in particular estrogen response DNA elements (EREₛ). Moreover, the solvent selected and Vitamin E synergistically help the intracellular carrier transport the SERM by decreasing cellular and tissue inflammation and increase the bioavailability of the SERM as it is delivered to urogenital tissue.

The compositions may also include a minimal amount of lemon oil or peppermint oil. The compositions as used in the working examples below have 130 ml of liquid ingredients. The lemon oil and/or peppermint oil may be added thereto, in total, in an amount of 2-6 drops, which is about 0.1 ml to about 0.3 ml.

In operation, the composition reverses much of the urogenital atrophy of the urogenital cells so that dryness and inflammation are reduced and homeostasis and integrity return to the urogenital tissue. This allows women using the composition, as directed, to return to pleasant and non-painful intercourse, as supported by the Study included herein as Example 3. The composition also relieves vaginal pain associated with atrophic vaginitis, as well as decreases itching, vaginal dryness, vaginal bleeding and mild spotting, and the occurrence of urogenital infections.

In another aspect, compositions for topical administration in a urogenital area and/or a vagina for treatment of atrophic vaginitis, peri- and post-menopausal dyspareunia, and/or oopherectomized females prior to menopause that are free of estrogen and free of a selective estrogen receptor modulator are also disclosed. In other words, a sexual lubricant composition is disclosed that has similar compositions to those disclosed above, but without the SERM and without estrogen (i.e., they are estrogen free). The sexual lubricant composition is useful for vaginal or anal sex. Vaginal application of the sexual lubricant composition has shown some of the same beneficial effects on atrophic vaginitis, peri- and post-menopausal dyspareunia, and/or oopherectomized females prior to menopause as the compositions disclosed above.

The sexual lubricant composition may comprise any of the substances discussed above, including the additives. In its simplest form, the composition has a therapeutically effective amount of a medicinal grade honey, a therapeutically effective amount of a natural oil, and is free of estrogen and free of selective estrogen receptor modulators. The natural oil may be one or more of almond oil, avocado oil, coconut oil, corn oil, flaxseed oil, mustard oil, olive oil, peanut oil, rice bran oil, soybean oil, walnut oil, or other natural oils. The natural oil is preferably about 15% by volume to about 54% by volume of the composition. In one embodiment, the natural oil is avocado oil, coconut oil, and/or olive oil. In another embodiment, the natural oil is a 1:1 mixture of avocado oil and coconut oil. The medicinal grade honey includes Manuka honey, and is about 30% by volume to about 62% by volume of the composition. The composition may include one or more of the thickening agents, one or more tocopherol and/or tocotrienols that have Vitamin E activity, one or more pain relievers, one or more pH adjusters, and one or more additives discussed above.

The sexual lubricant comprises ingredients that will not deteriorate the material of a condom, including non-latex condoms.

### Method of Making

The compositions for topical vaginal application may be made by warming and mixing the intracellular carrier, the cellular anti-inflammatory agent, and the tocopherols and/or tocotrienols. In one embodiment, the warming of the components may be performed using a double boiler. This may be within a temperature range ; about 35°C (95°F) to about 49°C (120°F). This portion of the composition is referred to as Phase A. Phase B of the composition includes the primary active agent, the SERM, in powder form dissolved in a solvent. The method may include grinding the primary active agent into a powder before dissolving in the solvent.

Next, the warmed ingredients of Phase A are mixed with the dissolved primary active agent of Phase B to form the composition. In one embodiment, the composition is an emulsion, which may have a cream consistency for topical application of the composition. In another embodiment, the composition may be in the form of a suppository or a softgel encapsulating the composition. The softgel may be a gelatin based shell filled with the composition using encapsulation processes known in the art. The softgel shell may be a combination of gelatin, water, and a plasticizer such as glycerin and/or sorbitol(s) or other polymer such as starch and carrageenan rather than gelatin.

A thickening agent, if present, may be added to Phase B of the composition before mixing with Phase A, or may be added to the composition after Phase A and Phase B are mixed together. If needed, the pH of the composition may be adjusted by adding a pH adjuster thereto with mixing. The pH of the composition is preferably in a range of about 4.5 to about 6. Accordingly, enough pH adjuster is added to the composition to adjust the pH to be within this range.

If a pain reliever is present in the composition, it is preferably added into the solvent in Phase A before Phase A is mixed with Phase B. Other additives may be added to either Phase A or Phase B as appropriate or even to the composition after Phase A and Phase B are mixed together.

The method of making the compositions that are free of estrogen and free of SERM includes carrying out just Phase A above. If needed, the pH of the composition may be adjusted by adding a pH adjuster to Phase A with mixing. The pH of the composition is preferably in a range of about 4.5 to about 6. Accordingly, enough pH adjuster is added to the composition to adjust the pH to be within this range. If a pain reliever or other additives are present, they are also added to Phase A.

### Method of Treatment

A composition according to the invention is also claimed for use in treating atrophic vaginitis : by topical application in the vagina. About 2 ml to about 8 ml of the composition is topically applied to the urogenital area and/or into the vagina. The topical application may be daily, every other day, or at least once weekly. In one embodiment, the composition is topically applied as about 3 ml to about 5 ml to the urogenital area and/or into the vagina.

The topical application of the composition is preferably performed just prior to the user lying down to rest for at least about four hours. In one embodiment, the topical application of the composition is performed just prior to lying down to go to sleep, and is typically performed once daily, but is not limited thereto.

The dosage for the SERM free compositions is the same as noted above when the SERM is present.

### Example 1: Vaginal Cream/Emulsion

**Table 1: Formula 1**

| **Component** | **Example** | **Amount** | **% Volume** |
|---|---|---|---|
| Phase A | | | |
| Intracellular Carrier | Avocado oil | 40 ml | 31% |
| Cellular Anti-inflammatory Agent | Manuka Honey | 60 ml | 46% |
| Tocopherol | Tocopherol acetate | 20 ml | 15.2% |
| (optional) | lemon oil or peppermint oil | 0.1-0.3 ml | at most 0.2% |

| Phase B | | | |
|---|---|---|---|
| Solvent | Glycerin | 10 ml | 7.6% |
| SERM | Raloxifene | 90 mg | n/a (solid) |

The avocado oil, the Manuka honey, and the tocopherol were warmed in a double boiler until the temperature was about 35°C (95°F) to about 49°C (120°F) to make Phase A. The 90 mg of raloxifene was ground into a powder, and thereafter dissolved in the glycerin to make Phase B. The warmed ingredients of Phase A were blended with the dissolved raloxifene of Phase B to form an emulsion having a pH of about 6. The emulsion was stored in a glass container at room temperature away from sunlight.

### Example 2: Vaginal Cream/Emulsion

**Table 2: Formula 2**

| **Component** | **Example** | **Amount** | **% Volume** |
|---|---|---|---|
| Intracellular Carrier | Avocado oil | 40 ml | 31% |
| Cellular Anti-inflammatory Agent | Manuka Honey | 60 ml | 46% |
| Solvent | Glycerin | 10 ml | 7.7% |
| Tocopherol | Tocopherol acetate | 10 ml | 7.7% |
| Thickening Agent | Propylene Glycol | 10 ml | 7.7% |
| SERM | Raloxifene | 60 mg | n/a (solid) |

The avocado oil, the Manuka honey, and the tocopherol were warmed in a double boiler until the temperature was about 35°C (95°F) to about 49°C (120°F) to make Phase A. The 60 mg of raloxifene was ground into a powder, and thereafter dissolved in the glycerin and propylene glycol to make Phase B. The warmed ingredients of Phase A were mixed or blended with the dissolved raloxifene of Phase B to form an emulsion having a pH of about 6. The emulsion was stored in a glass container at room temperature away from sunlight.

### Example 3: Study

Ten women participated in a volunteer study to compare the effectiveness of the composition of Example 2 against a representative formulation from U.S. Patent No. 5,610,167, in particular the topical composition of formulation 10, set forth below, but modified to include the same amount of raloxifene as present in the formulation of Example 2. The amount of the active ingredient was adjusted to be the same so that the difference in performance of the two formulations, in particular the carrier and other ingredients, is shown to contribute significantly to the performance of the formulations disclosed herein.

**Table 3: Prior Art: Formulation 10**

| | |
|---|---|
| hydroxypropyl cellulose | 1.5 g |
| ethyl lactate | 15.0 g |
| Active ingredient (raloxifene) | 60 mg* |
| isopropanol qs | 100 g |

| | |
|---|---|
| *amount of active equal to the amount of active in Example 2 | |

Five women were give formulation 10 (Group I), reproduced above, with instructions to apply 4 ml of formulation 10 onto the urogenital area and into the vagina daily generally proximate to bedtime, i.e., a period of lying down for at least four hours. The other five women were given the formulation of Example 2 (Group II) above with instructions to apply 4 ml of the emulsion into the vagina daily generally proximate to bedtime, i.e., a period of lying down for at least four hours. The study was conducted over a five-week period with weekly follow-up consultations.

Group I included women between the ages of 45 and 60, all of which are experiencing menopause or peri-menopause. The younger women were experiencing early menopause as a result of cancer treatments and/or the removal of the ovaries. Three out of the five women in Group I were told by a health care provider that they cannot use products that include estrogen and the other two expressed a fear of using hormones. All the women experienced urinary incontinence and all the women were diagnosed with atrophic vaginitis. Each woman attested to pain with intercourse, vaginal pain and/or itching, vaginal dryness, and vaginal bleeding and/or mild spotting.

**Table 4:**

| **Week** | **Assessment** |
|---|---|
| 1 | Women reported no relief from vaginal or urinary symptoms, unpleasant side effects of itching and burning with the application of the formulation, and no attempt at intercourse. |
| 2 | Three women reported slight relief from vaginal dryness, all women reported unpleasant side effects of itching and burning with the application of the formulation and no attempt at intercourse. |
| 3 | All women reported some slight relief from vaginal dryness and vaginal pain, but continued itching and burning upon application, such that one woman stopped using the formulation. Again, no intercourse for any of the women. |
| 4 | The four women remaining reported slight relief from vaginal or urinary symptoms, but continued itching, burning, and irritation upon application. There was no intercourse for any of the women, but two reported that the area could be touched without pain. |
| 5 | Another women stopped using the formulation. The remaining three women still reported slight improvement of vaginal and urinary symptoms, but continued itching, burning, and irritation upon application. No intercourse, but digital caressing without pain was possible, with minimal digital penetration for two women with minimal discomfort. |

Group II included women between the ages of 34 and 70, all of which are experiencing menopause or peri-menopause. The younger women were experiencing menopause as a result of cancer treatments and/or the removal of the ovaries. Four out of the five women in Group II were told by a health care provider that they cannot use products that include estrogen. Four out of the five women experienced urinary incontinence and all the women were diagnosed with atrophic vaginitis. Each woman attested to pain with intercourse, vaginal pain and/or itching, vaginal dryness, and vaginal bleeding and/or mild spotting.

**Table 5:**

| **Week** | **Assessment** |
|---|---|
| 1 | Women reported relief from vaginal or urinary symptoms between days 4-7. No intercourse for any of the women. |
| 2 | Each of the women reported continued daily improvement of vaginal or urinary symptoms, including reduced itching, dryness, and pain. Two of the women attempted intercourse; both reported slight initial discomfort with penetration. |
| 3 | All women reported additional improvement of vaginal or urinary symptoms. Three women reported no intercourse, but two reported digital foreplay without pain and one reported digital penetration without pain. Two women were able to have intercourse, with at least partial penetration several times without pain. |
| 4 | All women reported additional improvement of vaginal or urinary symptoms, including daily relief from itching, dryness, pain, and including less urine loss. All women attempted intercourse, with at least digital penetration of 1-3 inches without pain. Continually less pain during intercourse, with increased moisture and elastic feel. |
| 5 | All women reported continuous relief from vaginal or urinary symptoms, including less urges to urinate frequently. All women were able to have intercourse with at least 1-3 inches of penile penetration without pain. |

According to the study, the composition of Example 2, disclosed herein, was much more effective than formulation 10 from U.S. Patent No. 5,610,167. Here, the women experienced improvement of symptoms of atrophic vaginitis (reduced symptoms) within 4-7 days and continual improvement over the five weeks. Even more important to the women and their spouses or partners was the improvement in their sex life - penetration without pain, increased moisture, and elastic feel to the tissue. Further, some women even reported an improvement in their libido, which further enhanced their sex life.

### Example 4: SERM Free - Vaginal Cream/Emulsion

**Table 6: Formula 3**

| **Composition A** | | | |
|---|---|---|---|
| **Component** | **Example** | **Amount** | **% Volume** |
| Natural Oil | Avocado Oil | 40 ml | 33.4% |
| Cellular Anti-inflammatory Agent | Manuka Honey | 60 ml | 50% |
| Tocopherol | Tocopherol acetate | 10 ml | 8.3% |
| Thickening Agent | Propylene Glycol | 10 ml | 8.3% |

**Table 7: Formula 4**

| **Composition B** | | | |
|---|---|---|---|
| **Component** | **Example** | **Amount** | **% Volume** |
| Natural Oil | Avocado Oil | 20 ml | 16.7% |
| Natural Oil | Coconut Oil | 20 ml | 16.7% |
| Cellular Anti-inflammatory Agent | Manuka Honey | 60 ml | 50% |
| Tocopherol | Tocopherol acetate | 10 ml | 8.3% |
| Thickening Agent | Propylene Glycol | 10 ml | 8.3% |

## Claims

1. A composition for topical administration in a urogenital area and/or a vagina comprising:
a therapeutically effective amount of a selective estrogen receptor modulator (SERM), wherein the SERM comprises 0.038 mg/ml of the composition to 1.9 mg/ml of the composition;
an intracellular carrier for carrying the SERM into a cell, wherein the intracellular carrier is a natural oil; and
a therapeutically effective amount of a cellular anti-inflammatory agent, wherein the cellular anti-inflammatory agent comprises a medicinal grade honey as 30% by volume to 62% by volume of the total liquid ingredients.

2. The composition of claim 1, wherein the SERM is selected from the group consisting of droloxifene, idoxifene, raloxifene, tamoxifen, toremifene, TAT-59, and combinations thereof.

3. The composition of claim 1, wherein the SERM comprises raloxifene.

4. The composition of claim 1, wherein the intracellular carrier comprises a natural oil selected from the group consisting of almond oil, avocado oil, coconut oil, corn oil, flaxseed oil, mustard oil, olive oil, peanut oil, rice bran oil, soybean oil, walnut oil, and combinations thereof.

5. The composition of claim 1, wherein the intracellular carrier comprises one or more of avocado oil, coconut oil, and olive oil.

6. The composition of claim 1, wherein the intracellular carrier comprises about 15% by volume to 54% by volume of the liquid ingredients.

7. The composition of claim 1, further comprising a solute that dissolved the SERM.

8. The composition according to claim 1 for the use in the treatment of atrophic vaginitis, peri- and post-menopausal dyspareunia, and/or oopherectomized females prior to menopause, wherein about 2 ml to about 8 ml of the composition is applied topically to the urogenital area and/or into the vagina daily, every other day, or at least once weekly.

9. The composition of claim 1, **characterized by** being free of estrogen.

10. The composition of claim 9, wherein the natural oil is selected from the group consisting of almond oil, avocado oil, coconut oil, corn oil, flaxseed oil, mustard oil, olive oil, peanut oil, rice bran oil, soybean oil, walnut oil, and combinations thereof and the natural oil is about 15% by volume to about 54% by volume of the composition.

11. The composition of any of claims 1 to 9, 10 and the composition for the use according to claim 8, wherein the medicinal grade honey includes Manuka honey.

12. The composition of any of claims 1 to - 7, 9-11 and the composition for the use according to claim 8, further comprising a thickening agent, and one or more tocopherol and tocotrienols that have Vitamin E activity.

## Patentansprüche

1. Zusammensetzung zur topischen Verabreichung in einem urogenitalen Bereich und/oder einer Vagina, umfassend:
eine therapeutisch wirksame Menge eines selektiven Östrogenrezeptor-Modulators (SERM), wobei der SERM 0,038 mg/ml der Zusammensetzung bis 1,9 mg/ml der Zusammensetzung umfasst;
einen intrazellulären Träger zum Transportieren des SERM in eine Zelle, wobei der intrazelluläre Träger ein natürliches Öl ist; und
eine therapeutisch wirksame Menge eines zellulären entzündungshemmenden Mittels, wobei das zelluläre entzündungshemmende Mittel 30 Volumenprozent bis 62 Volumenprozent der gesamten flüssigen Bestandteile an Honig von medizinischer Qualität umfasst.

2. Zusammensetzung gemäß Anspruch 1, wobei der SERM ausgewählt ist aus der Gruppe bestehend aus Droloxifen, Idoxifen, Raloxifen, Tamoxifen, Toremifen, TAT-59 und Kombinationen davon.

3. Zusammensetzung gemäß Anspruch 1, wobei der SERM Raloxifen umfasst.

4. Zusammensetzung gemäß Anspruch 1, wobei der intrazelluläre Träger ein natürliches Öl umfasst, das ausgewählt ist aus der Gruppe bestehend aus Mandelöl, Avocadoöl, Kokosnussöl, Maisöl, Leinsamenöl, Senföl, Olivenöl, Erdnussöl, Reiskleieöl, Sojabohnenöl, Walnussöl und Kombinationen davon.

5. Zusammensetzung gemäß Anspruch 1, wobei der intrazelluläre Träger eines oder mehrere von Avocadoöl, Kokosnussöl und Olivenöl umfasst.

6. Zusammensetzung gemäß Anspruch 1, wobei der intrazelluläre Träger etwa 15 Volumenprozent bis etwa 54 Volumenprozent der flüssigen Bestandteile umfasst.

7. Zusammensetzung gemäß Anspruch 1, ferner umfassend einen gelösten Stoff, der den SERM auflöst.

8. Zusammensetzung gemäß Anspruch 1 zur Verwendung bei der Behandlung von atrophischer Vaginitis, peri- und postmenopausaler Dyspareunie und/oder oopherektomierten Frauen vor der Menopause, wobei etwa 2 ml bis etwa 8 ml der Zusammensetzung täglich, jeden zweiten Tag oder mindestens einmal wöchentlich topisch auf den urogenitalen Bereich und/oder in die Vagina aufgetragen werden.

9. Zusammensetzung gemäß Anspruch 1, **dadurch gekennzeichnet, dass** sie frei von Östrogen ist.

10. Zusammensetzung gemäß Anspruch 9, wobei das natürliche Öl ausgewählt ist aus der Gruppe bestehend aus Mandelöl, Avocadoöl, Kokosnussöl, Maisöl, Leinsamenöl, Senföl, Olivenöl, Erdnussöl, Reiskleieöl, Sojabohnenöl, Walnussöl und Kombinationen davon, und das natürliche Öl etwa 15 Volumenprozent bis etwa 54 Volumenprozent der Zusammensetzung ausmacht.

11. Zusammensetzung gemäß mindestens einem der Ansprüche 1 bis 7, 9, 10 und die Zusammensetzung zur Verwendung gemäß Anspruch 8, wobei der Honig von medizinischer Qualität Manuka-Honig einschließt.

12. Zusammensetzung gemäß mindestens einem der Ansprüche 1 bis 7, 9 bis 11 und die Zusammensetzung zur Verwendung gemäß Anspruch 8, ferner umfassend ein Verdickungsmittel und ein oder mehrere Tocopherol und Tocotrienole mit Vitamin-E-Aktivität.

## Revendications

1. Composition pour administration topique dans une zone urogénitale et/ou un vagin comprenant :
une quantité thérapeutiquement efficace d'un modulateur du récepteur d'œstrogènes sélectif (SERM), dans laquelle le SERM comprend de 0,038 mg/ml de la composition à 1,9 mg/ml de la composition ;
un vecteur intracellulaire pour transporter le SERM dans une cellule, dans laquelle le vecteur intracellulaire est une huile naturelle ; et
une quantité thérapeutiquement efficace d'un agent anti-inflammatoire cellulaire, dans laquelle l'agent anti-inflammatoire cellulaire comprend un miel de qualité médicinale représentant 30 % en volume à 62 % en volume des ingrédients liquides totaux.

2. Composition selon la revendication 1, dans laquelle le SERM est sélectionné dans le groupe constitué par le droloxifène, l'idoxifène, le raloxifène, le tamoxifène, le torémifène, TAT-59 et des combinaisons de ceux-ci.

3. Composition selon la revendication 1, dans laquelle le SERM comprend le raloxifène.

4. Composition selon la revendication 1, dans laquelle le vecteur intracellulaire comprend une huile naturelle sélectionnée dans le groupe constitué par l'huile d'amande douce, l'huile d'avocat, l'huile de coco, l'huile de maïs, l'huile de lin, l'huile de moutarde, l'huile d'olive, l'huile d'arachide, l'huile de riz, l'huile de soja, l'huile de noix, et des combinaisons de celles-ci.

5. Composition selon la revendication 1, dans laquelle le vecteur intracellulaire comprend une ou plusieurs parmi l'huile d'avocat, l'huile de coco, et l'huile d'olive.

6. Composition selon la revendication 1, dans laquelle le vecteur intracellulaire représente environ 15 % en volume à 54 % en volume des ingrédients liquides.

7. Composition selon la revendication 1, comprenant en outre un soluté où le SERM est dissous.

8. Composition selon la revendication 1 pour son utilisation dans le traitement de la vaginite atrophique, de la dyspareunie péri- et post-ménopausique, et/ou de femmes ovariectomisées avant la ménopause, dans laquelle environ 2 ml à environ 8 ml de la composition sont appliqués par voie topique à la zone urogénitale et/ou dans le vagin quotidiennement, tous les 2 jours, ou au moins une fois par semaine.

9. Composition selon la revendication 1, **caractérisée en ce qu'**elle est dépourvue d'œstrogènes.

10. Composition selon la revendication 9, dans laquelle l'huile naturelle est sélectionnée dans le groupe constitué par l'huile d'amande douce, l'huile d'avocat, l'huile de coco, l'huile de maïs, l'huile de lin, l'huile de moutarde, l'huile d'olive, l'huile d'arachide, l'huile de riz, l'huile de soja, l'huile de noix, et des combinaisons de celles-ci et l'huile naturelle représente environ 15 % en volume à environ 54 % en volume de la composition.

11. Composition selon l'une quelconque des revendications 1 à 7, 9, 10 et composition pour son utilisation selon la revendication 8, dans laquelle le miel de qualité médicinale comprend du miel Manuka.

12. Composition selon l'une quelconque des revendications 1 à 7, 9-11 et composition pour son utilisation selon la revendication 8, comprenant en outre un agent épaississant, et un ou plusieurs tocophérol et tocotriénols qui ont une activité de vitamine E.
